# EUROPÄISCHE PATENTANMELDUNG

(11) **EP 3 613 533 A1**
(43) Veröffentlichungstag der Anmeldung: **26.02.2020**
(21) Anmeldenummer: 18190805.4
(22) Anmeldetag: 24.08.2018
(51) Int. Cl.: B23K 26/08, B23K 26/364, B23K 26/0622, A61F 2/91, B23K 101/06, B23K 103/08

(54) **VERFAHREN ZUR HERSTELLUNG EINES IMPLANTATS SOWIE DURCH DAS VERFAHREN HERGESTELLTES IMPLANTAT**

(71) Anmelder: BIOTRONIK AG, 8180 Bülach (CH)
(72) Erfinder: REMUND, Stefan, 3005 Bern (CH); KRAMER, Thorsten, 4500 Solothurn (CH); NEUENSCHWANDER, Beat, 3400 Burgdorf (CH); JÄGGI, Beat, 3427 Utzenstorf (CH)
(74) Vertreter: Galander, Marcus

(57) **Zusammenfassung**

Die Erfindung betrifft ein Verfahren zur Herstellung eines Implantats, insbesondere Stents, aufweisend die Schritte:
Bereitstellen eines Implantatrohlings, insbesondere eines Stentvorformlings, aus einem degradierbaren Material, insbesondere einer Magnesium-Legierung; Einbringen von Ausnehmungen in den Implantatrohling, insbesondere Stentvorformling, durch Laserstrahlabtragen, wobei erfindungsgemäß der Implantatrohling, insbesondere Stentvorformling, zum Einbringen der Ausnehmungen mit Laserstrahlung in Form von Pulsbündeln (PB) bestrahlt wird, wobei jedes Pulsbündel (PB) zumindest zwei Pulse (P) aufweist.

## Beschreibung

Die vorliegende Erfindung betrifft ein Verfahren zur Herstellung eines Implantats, insbesondere eines Stents, aus einem degradierbaren Material, insbesondere aus einer Magnesium-Legierung, sowie einen durch das Verfahren hergestelltes Implantat, insbesondere Stent, aus einem solchen Material, insbesondere einer solchen Legierung. Die vorliegende Erfindung wird hauptsächlich am Beispiel eines Verfahrens zur Herstellung eines Stents aus einer Magnesium-Legierung beschrieben. Die vorliegende Erfindung ist jedoch nicht auf diese Anwendung eingeschränkt sondern zur Herstellung eines Implantats aus einem beliebigen degradierbaren Material geeignet. Unter einem degradierbaren Material wird im Rahmen dieser Anwendung ein Material verstanden, welches im Körper abgebaut wird (degradiert). Implantate aus degradierbaren Material (biodegradierbare Implantate) halten dabei über einen Zeitraum von Stunden/Tagen über Monate bis einige Jahre ihre strukturelle Integrität aufrecht und werden dann vom Körper abgebaut (resorbiert). Derartige degradierbare Materialien sind dem Fachmann bekannt, beispielsweise einige metallische Legierungen wie Magnesium-, Eisen- oder Zinklegierungen oder abbaubare Polymere wie Polylaktide.

Bei derartigen Herstellungsverfahren wird zum Schneiden des Stents oftmals Laserstrahlung eingesetzt, wobei insbesondere gepulste Laserstrahlung zum Materialabtragen/Schneiden verwendet wird.

Die Pulse werden durch die Pulsdauer und ihre Pulsenergie beschrieben. Die Pulsdauer wird typischerweise als Full Width at Half Maximum (FWHM) angegeben, wobei die Breite des Pulses bei der halben Pulshöhe gemessen wird. Für ultrakurze Pulse mit Pulsdauern im Bereich einiger Femtosekunden bis einige Pikosekunden kann die Pulsform als zeitlich symmetrisch angesehen werden.

Die Energie eines Pulses wird formal durch die Integration des zeitlichen Verlaufs der Pulsleistung (die Pulsform) über die Zeit berechnet und wird in Joule (J), Millijoule (mJ) oder Mikrojoule (µJ) angegeben. Sie kann ebenfalls als gemittelter Wert aus dem Quotienten der mittleren Leistung und der Repetitionsrate berechnet werden.

Die Repetitionsrate oder auch Pulsfolgefrequenz legt dabei fest, wie viele Einzelpulse pro Zeiteinheit vom Laser ausgesandt werden. Sie wird in Hertz (Hz), Kilohertz (kHz) oder Megahertz (MHz) angegeben.

Anstelle eines Einzelpulses ist es bei einigen Laserstrahlquellen möglich, bei gegebener Repetitionsrate die Pulsenergie eines Einzelpulses auf eine Folge von einzelnen Pulsen, sogenannte Pulsbündel oder Pulse Bursts, aufzuteilen.

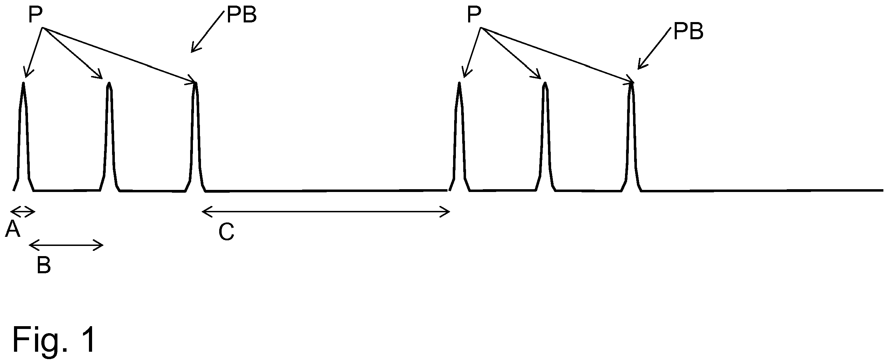

Ein Pulsbündel oder Pulse Burst (PB) ist eine Folge von einzelnen Pulsen, die aus mindestens zwei aufeinanderfolgenden einzelnen Pulsen (P) besteht, wobei der zeitliche Abstand (B) der einzelnen Pulse eines Pulsbündels kleiner ist als der zeitliche Abstand (C) zweier Pulsbündel, d.h. der reziproke Wert der Repetitionsrate des Lasers. (Fig. 1). Bei einem Pulsbündel ist der zeitliche Abstand zwischen je zwei benachbarten Pulsen eines Pulsbündels kleiner als der zeitliche Abstand zwischen dem letzten Puls eines Pulsbündels und dem ersten Puls des nächsten Pulsbündels. Im Rahmen dieser Anmeldung werden die Begriffe Pulsbündel und Pulse Burst synonym verwendet.

Abhängig vom Aufbau des Lasers, wird bei ultra-kurzgepulsten Laserstrahlquellen zwischen Master-Oscillator-Power-Amplifier (MOPA) Systemen und Regenerative Amplifier (Regen) Systemen unterschieden. Die Erzeugung der Pulsbündel für MOPA-Systeme erfolgt im so genannten Pulse Picker, der nicht nur einen einzelnen Puls, sondern mehrere Pulse (typ. 2 bis 10) mit der gewählten Repetitionsrate passieren lässt. Der zeitliche Abstand der Pulse im Pulsbündel entspricht demnach dem Kehrwert der Frequenz des Laser-Oszillators (Master Oscillator) und liegt typischerweise im Bereich von 20 MHz bis 100 MHz. Derartige Laserquellen erreichen heute bereits Frequenzen im GHz-Bereich. Die Oszillator-Frequenz liegt damit deutlich höher als typische Repetitionsraten von weniger als 1 MHz, die zurzeit für eine Bearbeitung eingesetzt werden. Vereinfacht ausgedrückt produziert die Laserquelle gepulste Laserstrahlung mit einer hohen Frequenz. Der Pulse Picker selektiert aus einer kontinuierlichen Folge von Pulsen die einzelnen Pulsbündel mit der gewünschten Repetitionsrate des Lasers und die einzelnen Pulse eines Pulsbündels. Damit legt der Pulse Picker die Repetitionsrate des Lasers (zeitlicher Abstand der Pulsbündel) und die Anzahl der Pulse eines Pulsbündels fest.

Die Einzelpulse oder Pulsbündel werden anschließend im Verstärker (Power Amplifier) verstärkt. Bei heutigen Ultrakurzpuls-Lasersystemen besteht dabei die Möglichkeit, den Verstärkungsfaktor für jeden Puls des Pulsbündels individuell einzustellen. So besteht insbesondere auch die Möglichkeit, die Verstärkung eines Pulses auf null einzustellen und den Puls damit auszublenden. Dadurch werden die individuelle Pulszahl und der zeitliche Abstand der individuellen Pulse eines Pulsbündels gesteuert. Im Prinzip kann so jedes Pulsbündel individuell gestaltet werden.

Ultrakurzpuls-Lasersysteme werden in der Regel zum Abtragen (Ablation) eingesetzt. Die Abtragrate bezeichnet dabei das pro Zeiteinheit abgetragene Volumen und wird typischerweise in Kubikmillimetern pro Minute (mm³/min) angegeben.

Unter der Abtrageffizienz oder der spezifischen Abtragrate wird die auf die eingebrachte mittlere Laserleistung bezogene Abtragrate verstanden, also das abgetragene Volumen pro Energieeinheit. Sie wird demnach in mm³/(min W) oder auch in **µ**m³/(**µ**J) angegeben.

Bei der Bearbeitung mit Laserstrahlung mit Einzelpulsen bei einer gewählten Repetitionsrate wird die maximale Abtrageffizienz u.a. durch die Entstehung von Konen und Krater auf der Werkstückoberfläche begrenzt, die die Absorption der Laserstrahlung nachteilig beeinflussen. Der Abtragprozess kann dadurch im schlimmsten Fall komplett zum Erliegen kommen.

Der Einsatz von Einzelpulsen bei einer moderaten mittleren Leistung und bei nicht zu hohen Repetitionsraten führt in der Regel zu der bestmöglichen Oberflächenqualität bei fast allen untersuchten Materialien.

Generell ist es bei derartigen Laserstrahlabtragverfahren wünschenswert, bei gleichbleibender Oberflächenqualität die Abtragrate zu steigern. Darüber hinaus ist es sinnvoll und wünschenswert, gleichzeitig die Abtrageffizienz nicht zu verringern oder sogar noch zu steigern.

Eine erhöhte Abtragrate kann bei gleichbleibender Qualität aber nur dann erreicht werden, wenn die Repetitionsrate des Lasers und damit seine mittlere Leistung entsprechend erhöht werden. Eine solche Erhöhung ist insbesondere durch zwei Faktoren begrenzt. Einerseits ist die mittlere Leistung bei kommerziellen ultrakurz-gepulsten Laserstrahlquellen, die üblicherweise verwendet werden, auf ca. 100 W beschränkt und andererseits zeigen mehrere Untersuchungen, dass bei einer Steigerung der Repetitionsrate die nachfolgenden Pulse zunehmend z. B. durch so genanntes Shielding, d.h. die Wechselwirkung oder teilweise Abschirmung der Laserstrahlung eines Pulses mit dem bereits abgetragenen Material der vorangegangenen Pulse, beeinflusst werden und dadurch die Abtrageffizienz sinkt.

Der Erfindung liegt daher die Aufgabe zugrunde, ein Verfahren zur Herstellung eines Implantats, insbesondere eines Stents, aus einem degradierbaren Material, insbesondere einer Magnesium-Legierung, zu schaffen, das eine erhöhte Abtrageffizienz aufweist.

Dieses Problem wird durch ein Verfahren mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 wird ein Verfahren zur Herstellung eines Implantats, insbesondere eines Stents, offenbart, aufweisend die Schritte:
- Bereitstellen eines Implantatrohlings, insbesondere eines Stentvorformlings, aus einem degradierbaren Material, insbesondere einer Magnesium-Legierung,
- Einbringen von Ausnehmungen in den Implantatrohling, insbesondere den Stentvorformling, durch Laserstrahlabtragen, insbesondere derart, dass ein Implantat, insbesondere ein Stent, ausgebildet wird, der eine Vielzahl von Zellen aufweist, die insbesondere jeweils durch miteinander verbundene Streben des Stents begrenzt sind,
- wobei erfindungsgemäß das Implantat, insbesondere der Stentvorformling, zum Einbringen der Ausnehmungen mit Laserstrahlung in Form von Pulsbündeln bestrahlt wird, wobei jedes Pulsbündel zumindest zwei Pulse aufweist.

Es hat sich überraschender Weise beim Abtragen von Magnesium-Legierungen gezeigt, dass insbesondere auf die Interaktion aufeinanderfolgender Einzelpulse, die eine Verringerung der Abtrageffizienz oder eine Reduktion der Bearbeitungsqualität bedeutet, durch den Einsatz von Pulsbündeln (Pulse Bursts) reagiert werden kann. Die einzelnen Pulse innerhalb eines Pulsbündels können dabei individuell so in Höhe, also in der Energie jedes einzelnen Pulses, und zeitlichem Abstand (in Vielfachen des Kehrwerts der Frequenz des Laser-Oszillators) eingestellt werden, dass nicht nur der negative Einfluss kompensiert, sondern die Abtrageffizienz gesteigert werden kann. Da darüber hinaus die Repetitionsrate nicht im gleichen Umfang wie bei Einzelpulsen gesteigert werden muss, können die Interaktionen zwischen den einzelnen Pulsbündeln verringert oder sogar vollkommen vermieden werden.

Durch die angepasste Anwendung von Pulsbündeln beim Abtragen mit ultrakurz-gepulster Laserstrahlung insbesondere von Metallen kann die spezifische Abtragrate, auch Abtrageffizienz genannt, also das abgetragene Volumen pro Zeiteinheit und eingebrachter mittlerer Laserleistung, erhöht werden.

Beim Abtragen mit ultrakurz-gepulster Laserstrahlung treten verschiedene Effekte an der Werkstückoberfläche und darüber auf, die den Abtragprozess beeinflussen. Dies können insbesondere Modifikationen der Oberfläche oder Abschirmungen durch abgetragene Partikel sein. Durch gezielten Einsatz von Pulse Bursts in einer Konfiguration, die den Mechanismen, welche den Abtragprozess negativ beeinflussen, entgegenwirkt, kann bei Magnesium-Legierungen die Abtrageffizienz erhöht und sogar über den Wert eines einzelnen Pulses gesteigert werden.

Ein vergleichbarer Effekt stellt sich bei anderen degradierbaren Materialien ein, insbesondere bei degradierbaren Polymeren wie Polylaktide oder anderen metallischen Legierungen wie Eisen- oder Zinklegierungen ein.

Gemäß einer bevorzugten Ausführungsform ist vorgesehen, dass jedes Pulsbündel der zum Abtragen verwendeten Laserstrahlung drei Pulse aufweist, insbesondere genau bzw. lediglich drei Pulse. Diesbezüglich konnte gezeigt werden, dass bei einer Magnesium-Legierung gegenüber einem Einzelpuls der Einsatz eines Pulsbündels mit drei Pulsen die Abtrageffizienz von 0,19 mm³/(min•W) um 68% auf 0,32 mm³/(min•W) steigert.

Gemäß einer weiteren bevorzugten Ausführungsform ist vorgesehen, dass jedes Pulsbündel der zum Abtragen verwendeten Laserstrahlung vier Pulse aufweist, insbesondere genau bzw. lediglich vier Pulse. Diesbezüglich konnte gezeigt werden, dass bei einer Magnesium-Legierung gegenüber einem Einzelpuls der Einsatz eines Pulsbündels mit vier Pulsen die Abtrageffizienz von 0,19 mm³/(min•W) um 142% auf 0,46 mm³/(min•W) steigert.

Gemäß einer weiteren bevorzugten Ausführungsform ist vorgesehen, dass jedes Pulsbündel der zum Abtragen verwendeten Laserstrahlung fünf Pulse aufweist, insbesondere genau bzw. lediglich fünf Pulse. Diesbezüglich konnte gezeigt werden, dass bei einer Magnesium-Legierung gegenüber einem Einzelpuls der Einsatz eines Pulsbündels mit fünf Pulsen die Abtrageffizienz von 0,19 mm³/(min•W) um 195% auf 0,56 mm³/(min•W) steigert.

Weiterhin ist gemäß einer Ausführungsform der Erfindung vorgesehen, dass der zeitliche Abstand je zweier benachbarter Pulse eines Pulsbündels in einem Bereich von 10ps bis 100 ns, bevorzugt 10 ns bis 50 ns (100 MHz bis 20 MHz), liegt, und insbesondere 12 ns beträgt.

Der Einsatz von Pulsbündeln, bei denen benachbarte Pulse des Pulsbündels einen zeitlichen Abstand von ca. 12 ns aufweisen, führt mit Vorteil zu einem lokal und in der Tiefe begrenzten Aufschmelzen der Oberfläche des Materials des Implantatrohlings, insbesondere des Stentvorformlings, so dass bereits die ersten Unebenheiten und Inhomogenitäten ausgeglichen werden und die Entstehung von Konen und Krater verhindert wird.

Weiterhin ist gemäß einer Ausführungsform der Erfindung vorgesehen, dass die Pulsdauer des jeweiligen Pulses eines Pulsbündels im Bereich von 0.200 ps bis 20 ps liegt und insbesondere 10 ps beträgt. Ebenfalls vorteilhaft sind Pulsdauern für die jeweiligen Pulse eines Pulsbündels im Bereich von 0.2-0.5 ps, 0.8-0.9 ps, 6-12 ps oder von rund 20 ps.

Weiterhin ist gemäß einer Ausführungsform der Erfindung vorgesehen, dass die Wiederholrate der Pulsbündel (auch Repetitionsrate) im Bereich von 0.1 MHz bis 20 MHz liegt und insbesondere 500 kHz beträgt. Ebenfalls vorteilhaft sind Wiederholraten für die Pulsbündel im Bereich von 0.1-1 MHz, 2 MHz oder 8 MHz.

Weiterhin ist gemäß einer Ausführungsform der Erfindung vorgesehen, dass die Wellenlänge der Laserstrahlung im Infrarot-Bereich von 1000 nm bis 1500 nm, insbesondere 1026-1090 nm liegt. In einigen Ausgestaltungen kann es aber auch vorteilhaft sein, eine Laserquelle mit einer Laserstrahlung im Bereich von 513-545 nm oder 342-363 nm zu verwenden.

Weiterhin kann der Implantatrohling, insbesondere Stentvorformling, gemäß einer Ausführungsform rohrförmig ausgebildet sein und erstreckt sich dabei entlang einer Längsachse bzw. Zylinderachse.

Vorzugsweise wird der Implantatrohling, insbesondere Stentvorformling, gemäß einer Ausführungsform der Erfindung zur Ausbildung der Ausnehmungen unter Beaufschlagung mit Laserstrahlung um die Längsachse rotiert und/oder in Richtung der Längsachse bewegt.

Gemäß einer Ausführungsform der Erfindung ist es auch möglich (insbesondere als Alternative zu einer Bewegung des Implantatrohlings, insbesondere Stentvorformlings, entlang seiner Längsachse), dass die Laserstrahlung zur Ausbildung der Ausnehmungen entlang der Längsachse bewegt wird (z.B. kann ein das Laserlicht aussendender Kopf eines Schneidwerkzeuges entlang der Längsachse des Implantatrohlings, insbesondere Stentvorformlings, bewegt werden).

Gemäß einem weiteren Aspekt der vorliegenden Erfindung wird ein Implantat, insbesondere ein Stent, offenbart, der mittels des erfindungsgemäßen Verfahrens hergestellt worden ist.

Die vorliegende Erfindung ist besonders zum Schneiden und Strukturieren von Gefässstützen/Stents geeignet. Da die Bearbeitung schichtweise durch Materialabtrag erfolgt, kann grundsätzlich mit Vorteil jegliche Geometrie bearbeitet werden.

Hinsichtlich der Bearbeitung von Gefäßstützen/Stents bringt die erfindungsgemäße Effizienzsteigerung eine entscheidende Verkürzung der Bearbeitungsdauer bei gleichzeitiger Steigerung der Flexibilität gegenüber dem herkömmlichen thermischen Laserstrahlschneiden.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine schematische Darstellung von Pulsbündeln (Pulse Bursts) mit drei Pulsen; und
- Fig. 2: die Abtrageffizienz über der in das Material eingestrahlten Energie pro Fläche φ₀ bei einer erfindungsgemäßen Magnesium-Legierung sowie bei Pulsbündeln mit drei, vier sowie fünf Pulsen gegenüber einem Einzelpuls.

Die Figur 1 zeigt eine schematische Darstellung von Pulsbündeln (PB) mit drei Pulsen P wie sie zum Schneiden eines Stentvorformlings aus einer Magnesium-Legierung der hierin beschriebenen Art verwendet werden können. Der Pulsbündel-Betrieb des zum Abtragen/Schneiden verwendeten Lasers ist dadurch gekennzeichnet, dass die zeitlichen Abstände B zweier benachbarter Pulse P eines Pulsbündels PB kleiner sind als der zeitliche Abstand C zwischen dem letzen Puls P eines Pulsbündels PB und dem ersten Puls P des darauffolgenden Pulsbündels PB.

Erfindungsgemäß wird ein Laserstrahl mit derartigen Pulsbündels PB auf den Stentvorformling gerichtet und eine Vielzahl an Ausnehmungen werden aus dem insbesondere rohrförmigen Stentvorformling herausgeschnitten bzw. abgetragen, um insbesondere einen Stent mit einer Vielzahl an Zellen zu erhalten, die von Streben des Stents umrandet sind.

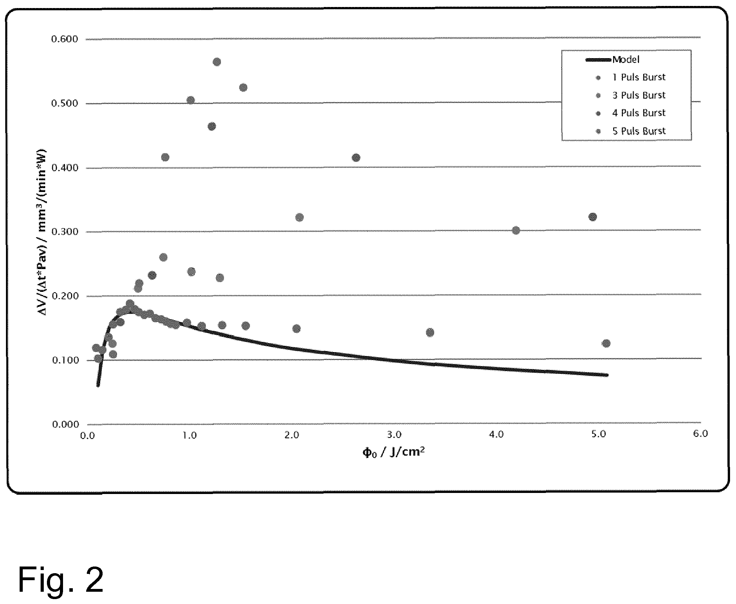

Wie in der Figur 2 dargestellt ist, kann die Abtrageffizienz, die auf der Y-Achse bzw. Ordinate aufgetragen ist (auf der Abszisse ist die in das Stentmaterial eingestrahlte Energie pro Fläche φ₀ aufgetragen), erhöht werden, wenn zum Abtragen des Stentmaterials (hier beispielsweise eine Magnesiumlegierung) Laserstrahlung mit Pulsbündeln mit drei, vier oder fünf Pulsen anstelle von Einzelpulsen verwendet wird.

Die hierbei verwendete Pulsdauer A der Pulse P der Pulsbündeln PB beträgt A = 10 ps. Der Abstand B der Pulse P beträgt B = 12 ns und der Abstand C der Pulsbündel PB beträgt C = 2 µs. Dies entspricht einer Repetitionsrate (Pulsfolgefrequenz) der Pulsbündel PB von 500 kHz. Es wird Laserstrahlung der Wellenlänge λ = 1064 nm verwendet (vgl. Fig. 1).

Zur Erzeugung der erfindungsgemäßen Pulsbündeln PB können grundsätzlich kommerzielle Lasersysteme verwendet werden, wie beispielsweise von LUMENTUM, Amplitude Systémes oder Coherent.

## Patentansprüche

1. Verfahren zur Herstellung eines Implantats, insbesondere eines Stents, aufweisend die Schritte:
- Bereitstellen eines Implantatrohlings, insbesondere ein Stentvorformlings, aus einem degradierbaren Material, insbesondere einer Magnesiumlegierung,
- Einbringen von Ausnehmungen in den Implantatrohling, insbesondere den Stentvorformling, durch Laserstrahlabtragen,
**dadurch gekennzeichnet,**
**dass** der Inplantatrohling, insbesondere der Stentvorformling, zum Einbringen der Ausnehmungen mit Laserstrahlung in Form von Pulsbündeln (PB) beaufschlagt wird, wobei jedes Pulsbündel (PB) zumindest zwei Pulse (P) aufweist.

2. Verfahren nach Anspruch 1, **dadurch gekennzeichnet, dass** jedes Pulsbündel (PB) durch drei Pulse (P) gebildet wird.

3. Verfahren nach Anspruch 1, **dadurch gekennzeichnet, dass** jedes Pulsbündel (PB) durch vier Pulse (P) gebildet wird.

4. Verfahren nach Anspruch 1, **dadurch gekennzeichnet dass** jedes Pulsbündel (PB) durch fünf Pulse (P) gebildet wird.

5. Verfahren nach einem der vorhergehenden Ansprüche, **dadurch gekennzeichnet, dass** der zeitliche Abstand (B) je zweier benachbarter Pulse (P) eines Pulsbündels (PB) in einem Bereich von 10 ps bis 100 ns liegt und insbesondere zwischen 10 ns und 50 ns beträgt.

6. Verfahren nach einem der vorhergehenden Ansprüche, **dadurch gekennzeichnet, dass** die Pulsdauer (A) des jeweiligen Pulses (P) eines Pulsbündels (PB) im Bereich von 0.200 ps bis 20 ps liegt und insbesondere 10 ps beträgt.

7. Verfahren nach einem der vorhergehenden Ansprüche, **dadurch gekennzeichnet, dass** die Repetitionsrate im Bereich von 0.1 MHz bis 20 MHz liegt und insbesondere 500 kHz bis 5 MHz beträgt.

8. Verfahren nach einem der vorhergehenden Ansprüche, **dadurch gekennzeichnet, dass** die Wellenlänge der Laserstrahlung im infrarot Bereich von 1000 nm bis 1500 nm, insbesondere 1026-1090 nm liegt.

9. Verfahren nach einem der vorhergehenden Ansprüche, **dadurch gekennzeichnet, dass** der Implantatrohling, insbesondere der Stentvorformling, rohrförmig ausgebildet ist und sich entlang einer Längsachse erstreckt.

10. Verfahren nach Anspruch 9, **dadurch gekennzeichnet, dass** der Implantatrohling, insbesondere der Stentvorformling, zur Ausbildung der Ausnehmungen unter Bestrahlung mit der Laserstrahlung um die Längsachse rotiert wird und/oder in Richtung der Längsachse bewegt wird.

11. Verfahren nach Anspruch 10, **dadurch gekennzeichnet, dass** die Laserstrahlung zur Ausbildung der Ausnehmungen entlang der Längsachse bewegt wird.

12. Implantat, insbesondere Stent, hergestellt mit einem Verfahren nach einem der Ansprüche 1 bis 11.
